# EUROPEAN PATENT APPLICATION

(11) **EP 4 104 881 A1**
(43) Date of publication of application: **21.12.2022**
(21) Application number: 21315096.4
(22) Date of filing: 17.06.2021
(51) Int. Cl.: A61M 5/20, A61M 5/315, F16D 27/00

(54) **DRUG DELIVERY DEVICE AND METHOD FOR OPERATING A DRUG DELIVERY DEVICE**

(71) Applicant: Sanofi, 75008 Paris (FR)
(72) Inventor: The designation of the inventor has not yet been filed
(74) Representative: Sanofi Aventis Deutschland GmbH

(57) **Abstract**

The present invention refers to a drug delivery device (1) and to a method of operating same. The drug delivery device (1) may comprise a housing (10; 32), a dial grip (12) manually operable by a user for setting an individual dose to be delivered by the drug delivery device (1), a dose setting and drive mechanism, which is configured to perform a dose setting operation in response to the operation of the dial grip (12) and which is configured to perform a dose delivery operation for delivering the set dose, a clutch (20) comprising two clutch members (21, 22; 31, 33; 43, 44) for rotationally coupling and de-coupling the dial grip (12) to the housing (10; 32) and/or to the dose setting and drive mechanism, at least one processor, a sensor arrangement (29, 30) connected to the at least one processor and operable to generate measurement data indicative of the dose setting operation, and a power source (28) connected to the at least one processor. The drug delivery device (1) further comprises a clutch actuator (23, 24; 23; 39, 23, 42) operable by the at least one processor to trigger a switch of the state of the clutch (20). The at least one processor is configured to operate the clutch actuator (23, 24; 23; 39, 23, 42) if the measurement data generated by the sensor arrangement (29, 30) reaches a threshold.

## Description

The present invention is generally directed to a drug delivery device. In more detail, the present invention relates to a manually operated variable dose drug delivery device comprising an electronic module suitable for detecting a dialled and/or dispensed dose amount and/or suitable for operation of a switch or clutch. Further, the present invention is directed to a method for operating such a drug delivery device.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is applicable for disposable and reusable devices.

For such devices the functionality of recording doses that are dialled and/or delivered from the pen may be of value to a wide variety of device users as a memory aid or to support detailed logging of dose history. Thus, drug delivery devices using electronics are becoming increasingly popular in the pharmaceutical industry as well as for users or patients.

For example, a drug delivery device is known from EP 2 814 545 A1 comprising an electronic clip-on module. The clip-on module comprises a battery which powers a processor and further components controlled by the processor, like light-sources, a photometer, an acoustic sensor, an acoustical signal generator and a wireless unit, like a Bluetooth^{®} transceiver configured to transmit and/or receive information to/from another device in a wireless fashion. Another example of a module for detecting dose setting and which may be attached to a drug delivery device is known from WO 2020/035406 A1. In contrast to that, WO 2019/101962 A1 discloses a module integrated into a drug delivery device. This module comprises a rotary encoder system for detecting the amount of a dose selected for dispensing.

The present invention is applicable to different types of drug delivery devices, including but not limited to drug delivery devices having a dial extension, e.g. a drug delivery device as disclosed in EP 2 890 434 B1 or EP 2 890 435 B1 in which a dose setting operation requires rotation of a dial grip which due to a threaded interface in the device winds or screws out of a housing thereby increasing the overall length of the device and in which dose dispensing requires pushing a portion of the device back into the housing, and drug delivery devices without dial extension, e.g. a spring driven drug delivery device as disclosed in WO 2016/055619 A1.

A common means of dispensing an injectable medicinal product is a drug delivery device having a variable dose injection device. Such variable dose injection devices have a maximum dose value which may be selected and dispensed by a user. However, a variable dose injection device permits selecting individual doses of a medicament to be dispensed up to the maximum dose value. Typically, a user selects an individual dose required due to a medicamentation scheme and/or in response to a health condition, e.g. the blood glucose level. When a drug delivery device platform is to be used for a number of injectable medicinal products, the designed maximum dose of the platform device may not be appropriate for each of the drugs to be delivered. One example may be long acting insulin and short acting insulin requiring different amounts of a maximum dose. Another example may be the use of a device for adults or for children requiring different maximum dose amounts. Thus, for some applications it may be advantageous to modifying the design of a suitably configured pen injector for the purpose of limiting the maximum dose that can be dialled and delivered from the pen. This functionality may be of value to a wide variety of device users to reduce the burden and risk of dose selection.

An example of a mechanical limitation of a maximum dose value to a reduced maximum dose is disclosed in EP 2 512 563 B1. The maximum settable dose of this variable dose device may be limited to a smaller value by inserting a maximum dose limiting sleeve which prevents selecting a dose exceeding a preselected dose value. Further, fixed dose devices are known which, in contrast to variable dose devices, permit only selecting one fixed dose value. These fixed dose and mechanically limited dose devices are currently known in the field but are restricted to set dose sizes during manufacture. Thus, a user is typically not able to change the amount of limitation. By contrast, a variable dose injection device offers good flexibility for users but requires significant engagement and every dose presents an opportunity for error. A fully automatic device could remove these risks but would require a significant increase in complexity, size, and cost of the device. Such drug delivery devices are typically manufactured in large scale such that an efficient and simple assembly is an important issue to keep production costs reasonably low.

Thus, it is an object of the present disclosure to provide a drug delivery device and a method of operating same requiring a much smaller impact on the device design, while still offering much of the benefit of full automation.

This object is solved for example by the subject matter defined in the independent claims. Advantageous embodiments and refinements are subject to the dependent claims. However, it should be noted that the disclosure is not restricted to the subject matter defined in the appended claims. Rather, the disclosure may comprise improvements in addition or as an alternative to the ones defined in the independent claims as will become apparent from the following description.

One aspect of the disclosure relates to a drug delivery device comprising a housing, a dial grip manually operable by a user for setting an individual dose to be delivered by the drug delivery device, a dose setting and drive mechanism, which is configured to perform a dose setting operation in response to the operation of the dial grip and which is configured to perform a dose delivery operation for delivering the set dose, a clutch comprising two clutch members for rotationally coupling and de-coupling the dial grip to the housing and/or to the dose setting and drive mechanism, at least one processor, a sensor arrangement, e.g. a dial encoder, connected to the at least one processor and operable to generate measurement data indicative of the dose setting operation, a power source connected to the at least one processor, and a clutch actuator operable by the at least one processor to trigger a switch of the state of the clutch. A switch of the state of the clutch is understood to be a switch from a state coupling the dial grip to the housing and/or to the dose setting and drive mechanism to de-coupling the dial grip from the housing and/or from the dose setting and drive mechanism or vice versa. The at least one processor may be configured to operate the clutch actuator if the measurement data generated by the sensor arrangement reaches an, e.g. preselected, threshold. Such a threshold may be a maximum dose value to be selected by a user of the drug delivery device. This has the effect of applying dynamic limits to a variable dose drug delivery device.

An independent aspect of the present disclosure is the provision of an electronically activated clutch in a manually operated drug delivery device of the variable dose type for limiting the maximum settable dose by either locking a dose dial grip of the drug delivery device to a stationary component part of the drug delivery device via actuation of the clutch or by decoupling the dose dial grip of the drug delivery device from further components of the dose setting and drive mechanism required for dose setting via actuation of the clutch. The electronically activated clutch may be a solenoid activated clutch comprising an electromagnetic coil and an actuator pin for operating the clutch, i.e. for switching the clutch between different clutch states, like coupling or de-coupling.

In other words, a user is allowed to dial to a predetermined or preselected dose value with minimal engagement or thought process. The user can dial a dose as normal, until the limited dose is reached as determined by the dial encoder, at which point further dialling is prevented by either locking and/or disengaging the dose selection mechanism. This is especially helpful in drug delivery devices in which dialling a dose requires rotation of the internal mechanism, i.e. the dial grip and/or the dose setting and drive mechanism, relative to the housing, e.g. an outer body. This rotation is generated by the user gripping the housing of the pen and applying torque to the dose setting and drive mechanism via the dial grip. Dialling can be prevented by either disconnecting the dial grip from the dose setting and drive mechanism or by locking the dial grip (directly or indirectly) to the housing. The user can then proceed to dispense the dose as normal, resetting the mechanism to allow for subsequent doses to be dialled.

Thus, it is an independent aspect of the present disclosure to lock or disengage the dose selection mechanism when the selected dose reaches a certain value. While the preferred method of locking and disengagement will be driven by the architecture of the base device, this approach could be applied to any variable dose injection device, especially but not limited to drug delivery devices as disclosed in EP 2 890 434 B1, EP 2 890 435 B1 or WO 2016/055619 A1.

The clutch actuator may comprise an electrical drive for translating at least one of the clutch members. An example for such a clutch actuator is a solenoid actuator comprising at least one electromagnet coil and an actuator pin which may comprise or may consist of a ferromagnetic material. In an example, the actuator pin may be secured to one of the clutch members, e.g. such that movement of the actuator pin causes or triggers a switch of the state of the clutch. For example, the at least one electromagnet coil may at least partially surround the actuator pin such that charging the at least one electromagnet coil with a first polarity generates a force biasing the actuator pin in a first direction, whereas charging the at least one electromagnet coil with a second polarity generates a force biasing the actuator pin in a second direction opposite to the first direction.

The clutch of the drug delivery device may be a bi-stable clutch. Such a bi-stable clutch may be held in two different states, i.e. coupled or de-coupled, without requiring a force applied by the user to maintain this state. Examples of holding a clutch in a stable state may include the provision of a spring and/or a magnet. For example, the clutch actuator may further comprise a permanent magnet secured on at least one of the clutch members. In more detail, the permanent magnet may be arranged such that it exerts a magnetic force on the actuator pin biasing the clutch members into contact. In an alternative example, a permanent magnet may be used to force the clutch members apart. In addition or as an alternative, the clutch actuator may further comprise a spring biasing the clutch members, e.g. in opposite directions. In a still further alternative, a spring may be provided biasing the clutch members into contact.

According to a further aspect of the present disclosure, the drug delivery device may be based on the working principles as disclosed e.g. in EP 2 890 434 B1 or EP 2 890 435 B1. Such a device or a further drug delivery device, e.g. with dial extension, may have the dial grip rotationally fixed to a first ring of teeth forming a first clutch member. Further, the actuator pin may be axially fixed to the first ring of teeth. Still further, the dose setting and drive mechanism may be rotationally fixed to a second ring of teeth forming a second clutch member. In this example, transmission of torque from the dial grip to the dose setting and drive mechanism may occur with the clutch coupled by engagement of the first ring of teeth with the second ring of teeth whereas transmission of torque from the dial grip to the dose setting and drive mechanism may be prevented with the clutch de-coupled by disengagement of the first ring of teeth with the second ring of teeth.

In other words, dose setting may be prevented at a given threshold with this configuration of a drug delivery device by disconnecting the dial grip from the dose setting and drive mechanism. Up to this threshold dose setting is allowed as usual, but after switching the state of the clutch torque can not be transferred to the dose setting and drive mechanism such that the user may continue to rotate the dial grip with the slipping clutch, however without further increasing the set dose.

In more detail, in an example the drug delivery device may comprise a bi-stable magnetic slip clutch connecting the dial grip to the dose setting and drive mechanism. This clutch may comprise a first clutch member in the form of a plate of the dose setting and drive mechanism and a second clutch member in the form of a plate connected to or part of the dial grip. The clutch can be locked into one of two positions open or closed. The plate of the dose setting and drive mechanism is held in a stable axial position relative to the housing. The plate of the dial grip (second clutch member) is able to move axially between a closed position, where it transfers torque to the dose setting and drive mechanism, and an open position, where the two plates (clutch members) are free to slip past each other. Rotating the dial grip can therefore either lead to dialling or slipping depending on the state of the clutch.

The clutch is switched between the two states by powering a latching solenoid clutch actuator. This clutch actuator operates by charging an electromagnetic coil in either polarity for a short time to generate a magnetic field. The field acts to move the actuator pin, on which the plate of the dial grip (second clutch member) is mounted, in either direction. In an exemplary embodiment, the latching behaviour may be generated by a permanent magnet situated within the plate of the dose setting and drive mechanism (first clutch member). A spring may hold the clutch plates apart, while the magnet and metal actuator pin draw them together. Since the spring force is linear, and the magnetic force varies with an inverse square law, a bi-stable system can be created with a switching point. If the clutch plates are positioned close together, the magnetic force will be greater than the spring force, and the plates will be drawn closed. If, however, they are in a position further apart than the switching point, they will be held apart by the spring. The electromagnet has sufficient strength to move the permanent magnet across the switching point from either direction.

Switching the clutch state from closed to open may include the following steps: The electromagnetic coil is powered generating a magnetic field which imparts a force on the actuator pin, pulling it away from the permanent magnet. Sufficient separation is achieved so that spring force is larger than the magnetic force of the permanent magnet. Then, the coil can be depowered. The spring pushes the clutch plate into the open position, such that the clutch teeth are disengaged or partially engaged, allowing for slipping. On the other hand, switching the clutch state from open to closed may include the following steps: The electromagnetic coil is powered generating a magnetic field which imparts a force on the actuator pin, pushing it towards the permanent magnet. Sufficient separation is achieved so that spring force is smaller than the magnetic force of the permanent magnet. Then, the coil can be depowered. The permanent magnet pulls the actuator pin into contact, such that the clutch teeth are fully engaged, transferring all torque from the dial grip to the dose setting and drive mechanism.

In an alternative, the permanent magnet could be replaced by a detent which is overcome in both directions by the solenoid and provides two stable positions.

In this example, an encoder may be used to detect relative rotation between the dial grip and the housing or any component part which is rotationally splined to the housing, e.g. a button to be pressed by a user for dose delivery. This encoder may function using an optical sensor and/or an electrical sensor, i.e. opening and closing electrical connections during said relative rotation.

According to a further aspect of the present disclosure, additionally, a detect switch may be used to monitor the position of the second clutch member, e.g. the plate of the dial grip. This switch may be positioned such that the clutch teeth cannot pass one another without the switch closing. If the dial grip is rotating and the detect switch is open, this will be counted as dialled units. If the dial grip is rotating, but the detect switch is closed, the processor will deduce that the clutch is slipping. With this encoder information, the processor can trigger a switch of clutch state when needed. If slipping is detected below the maximum dose limit, the electromagnet may be charged to engage the latching clutch. When the maximum dose limit is reached, the electromagnet may be charged to release the latch, allowing the clutch to slip.

Still further, a OU switch may be provided to reset the dialled dose count (and commit the dose record to memory). This can be achieved e.g. by shorting all the conductive strips in the OU (button pressed) position.

According to a further aspect of the present disclosure, wherein the drug delivery device may again be based on the working principles as disclosed e.g. in EP 2 890 434 B1 or EP 2 890 435 B1, the drug delivery device may comprise a stationary inner housing with splines forming a first clutch member, a dial clicker rotationally fixed to the dial grip, the dial clicker comprising an elastically deflectable arm for engaging the splines of the inner housing wherein the clicker arm forms a second clutch member, and a button axially displaceable relative to the dial clicker to perform the dose delivery operation for delivering the set dose. The button may comprise a trigger portion and an engagement portion wherein the engagement portion comprises a recess for receiving the elastically deflectable arm and axially spaced from the recess a blocking feature preventing deflection of the elastically deflectable arm. The actuator pin may be axially fixed to the engagement portion. With this configuration, a dose setting operation of the dial grip is permitted with the clutch de-coupled by positioning the recess such that deflection of the elastically deflectable arm is permitted thereby allowing relative rotation of the dial grip, whereas dose setting operation of the dial grip is blocked with the clutch coupled by preventing deflection of the elastically deflectable arm thereby preventing relative rotation of the dial grip. For example, the button may be a multi-component part with the trigger portion axially displaceable relative to the engagement portion by means of the actuator pin.

In other words, the dose setting may be prevented at a given threshold with this configuration of a drug delivery device by locking the dial grip directly or indirectly to the housing. Up to this threshold dose setting is allowed as usual, but after switching the state of the clutch further rotation of the dial grip in a direction increasing the set dose is prevented.

In more detail, in an example the drug delivery device may comprise a dial clicker, i.e. one or more component part(s) suitable for generating a clicking sound and/or a tactile feedback during dose setting. In this embodiment the dial grip may be mechanically splined to the dose setting and drive mechanism. The mode of operation is to lock the dial grip and/or the dose setting and drive mechanism directly or indirectly to the housing, e.g. to a stationary inner housing, via the dial clicker. In normal use, one or more dial clicker arms flex inward over splines on the inner housing on each unit of drug dialled, allowing for relative rotation. Flexing is permitted by allowing the arm(s) to enter into a recess upon deflection. The clicker arms are designed so that when the button is pressed down, they are blocked from flexing as the recess is axially shifted relative to the arm(s) during movement of the button. In this embodiment the button component may be split so that the lower portion can be moved down into the blocking position in advance of the upper portion. That is to say, dialling can be blocked prior to the dose button being pressed. A bi-stable solenoid actuator, similar to that described above, may be included in the button subassembly. In this way, the button effectively has two lengths. In the shorter configuration, the button acts in the same manner as the unmodified device, and the user can dial and dispense as normal. In the longer configuration, the user is unable to dial, and must proceed to dispense the dose.

In use, the solenoid will begin in the short configuration, and remain there until the encoder observes that the limit dose has been reached. At this point, the solenoid will be switched, driving the button into the longer state. The trigger portion of the button will be prevented from moving upward, meaning the engagement portion of the button must move down and block the dial clicker arms. The dial grip will then feel locked to the user. Pushing down on the button will dispense the dose and collapse the solenoid back into the shorter state. Therefore, once the dose is complete, and the button is released, the dial clicker arms will be unblocked once more and further doses can be dialled.

According to a still further aspect of the present disclosure, the dial grip may be rotationally fixed to a first ring of teeth forming a first clutch member (e.g. an axially displaceable collar), the actuator pin may be axially fixed to the first ring of teeth and the housing may be rotationally fixed to a second ring of teeth forming a second clutch member. With this configuration, the dose setting operation of the dial grip is permitted with the clutch de-coupled by disengaging the first ring of teeth from the second ring of teeth, whereas dose setting operation of the dial grip is blocked with the clutch coupled by engaging the first ring of teeth with the second ring of teeth.

Again, the dose setting may be prevented at a given threshold with this configuration of a drug delivery device by locking the dial grip directly or indirectly to the housing. Up to this threshold dose setting is allowed as usual, but after switching the state of the clutch further rotation of the dial grip in a direction increasing the set dose is prevented. Such a drug delivery device may be based on the working principle as disclosed in WO 2016/055619 A1.

In other words, similarly to the previous embodiment, the dial grip is mechanically splined to the dose setting and drive mechanism. The mode of operation may be to use a sliding collar to engage or disengage a locking clutch between the dose setting and drive mechanism and the housing. This embodiment may be best applied to devices without dial extension as disclosed in WO 2016/055619 A1 so that the button and housing remain at a fixed separation throughout dialling. Again, a bi-stable solenoid may be employed to provide the motion to switch the configuration of the button subassembly once the maximum encoder value is read.

In use, the solenoid will begin in the short configuration and hold the collar (first clutch member) .in clearance with the second clutch member formed by or on the housing. In this configuration dialling proceeds as with an unmodified device. Once the encoder reads that the limit dose has been reached, the solenoid will be switched, driving the collar into contact with the housing. The clutch teeth will prevent further dialling, but may be asymmetrically ramped to allow for dialling back down. This ramp would serve to overcome the bi-stable point of the solenoid, switching it back to the shorter configuration on a dialling down. Again, pushing down on the button will dispense the dose and collapse the solenoid back into the shorter state. Therefore, once the dose is complete and the button is released, the clutch will be open allowing further doses to be dialled.

The at least one processor, the sensor arrangement or dial encoder and the power source connected to the at least one processor may be part of an electronic module suitable for use with and/or in a drug delivery device. In other words, such a module may be integrated into the device or may be releasably attachable to the device. The electronic module preferably comprises a microcontroller as the processor, and may further comprise a communication unit with a wireless communication interface, at least one electronic user feedback generator, and/or a memory for storing measurement data.

If the electronic module is fitted with a small non-rechargeable, non-user replaceable coin cell, the overall power available over the life of the device is limited. Thus, the clutch actuator may be connected to an additional power source. As an alternative, the clutch actuator may be connected to the same power source as the processor.

The sensor arrangement may be connected to the at least one processor and operable to generate measurement data indicative of the dose setting operation and/or the dose delivery operation. The sensor arrangement may comprise one or more electrical switch(es) and/or may include optical and/or capacitive and/or acoustic sensors for detecting a movement of one or more component parts of the dose setting and drive mechanism of the drug delivery device. In one example, the sensor arrangement comprises at least one light source, e.g. an LED, and at least one light sensor, e.g. a photo detector. The sensor arrangement may be part of an encoding or motion sensing unit designed and working as described in unpublished EP 20315066.9 and EP 20315357.2, the disclosure of which is incorporated herein by reference.

The communication unit for communicating with another device may comprise a wireless communications interface for communicating with another device via a wireless network such as Wi-Fi or Bluetooth^{®}. In addition, the communication unit may comprise an interface for a wired communications link, such as a socket for receiving a Universal Series Bus (USB), mini-USB or micro-USB connector. Preferably, the electronic system comprises an RF, WiFi and/or Bluetooth^{®} unit as the communication unit. The communication unit may be provided as a communication interface between the module or the drug delivery device and the exterior, such as other electronic devices, e.g. mobile phones, personal computers, laptops and so on. For example, measurement data, i.e. dose data, may be transmitted by the communication unit to the external device. The dose data may be used for a dose log or dose history established in the external device.

The memory for storing measurement data, e.g. dose data, may be a separate memory or may be part of a main memory of the electronic module. These are controlled by the processor, which may for instance be the at least one microcontroller, a Digital Signal Processor (DSP), Application Specific Integrated Circuit (ASIC), Field Programmable Gate Array (FPGA) or the like. According to an aspect of the disclosure, the processor executes program code (e.g. software or firmware) stored in a program memory, and uses a main memory, for instance to store intermediate results, like dose data. Main memory may also be used to store a logbook on performed ejections/injections based on measurement data. Program memory may for instance be a Read-Only Memory (ROM), and main memory may for instance be a Random Access Memory (RAM).

According to a further aspect of the present disclosure, a method for operating a drug delivery device is provided. The drug delivery device may be a drug delivery device as described in one of the above aspects of the present disclosure. For example, the drug delivery device may comprise a housing, a dial grip manually operable by a user for setting an individual dose to be delivered by the drug delivery device, a dose setting and drive mechanism, which is configured to perform a dose setting operation in response to the operation of the dial grip and which is configured to perform a dose delivery operation for delivering the set dose, a clutch comprising two clutch members for rotationally coupling and de-coupling the dial grip to the housing and/or to the dose setting and drive mechanism, at least one processor, a sensor arrangement connected to the at least one processor and operable to generate measurement data indicative of the dose setting operation, a power source connected to the at least one processor, and a clutch actuator operable by the at least one processor to trigger a switch of the state of the clutch. For example, the method comprises the following steps:
a) setting an individual dose by manual operation of the dial grip,
b) generating measurement data indicative of the dose setting operation by the sensor arrangement,
c) operating the clutch actuator if the measurement data generated by the sensor arrangement reaches a threshold, thereby locking the dial grip or disengaging the dial grip from the dose setting and drive mechanism,
d) performing a dose delivery operation for delivering the set dose.

According to a further aspect, the present disclosure is directed to a computer program adapted to execute the above-mentioned method when implemented in a processor of an electronic module, e.g. the above-mentioned electronic module, the computer program comprising computer program means for: generating measurement data indicative of the dose setting operation by the sensor arrangement and operating the clutch actuator if the measurement data generated by the sensor arrangement reaches a threshold, thereby locking the dial grip or disengaging the dial grip from the dose setting and drive mechanism.

The drug delivery device may further comprise a container receptacle which is releasably attached to the dose setting and drive mechanism. As an alternative, the container receptacle may be permanently attached to the dose setting and drive mechanism. The container receptacle is adapted to receive a container, e.g. a cartridge, containing a medicament.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.
As described below, a drug or medicament can include at least one API, or combinations , thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.
The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.
The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.
Examples of APls for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.
Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide/ HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.
An example of an oligonucleotide is, for example: mipomersen sodium (Kynamro^{®}), a cholesterol-reducing antisense therapeutic for the treatment of familial hypercholesterolemia or RG012 for the treatment of Alport syndrom.
Examples of DPP4 inhibitors are Linagliptin, Vildagliptin, Sitagliptin, Denagliptin, Saxagliptin, Berberine.
Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.
Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.
The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.
The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.
Examples of antibodies are anti PCSK-9 mAb (e.g., Alirocumab), anti IL-6 mAb (e.g., Sarilumab), and anti IL-4 mAb (e.g., Dupilumab).
Pharmaceutically acceptable salts of any API described herein are also contemplated for use in a drug or medicament in a drug delivery device. Pharmaceutically acceptable salts are for example acid addition salts and basic salts.
Those of skill in the art will understand that modifications (additions and/or removals) of various components of the APls, formulations, apparatuses, methods, systems and embodiments described herein may be made without departing from the full scope and spirit of the present invention, which encompass such modifications and any and all equivalents thereof.
An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).
As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

The terms "axial", "radial", or "circumferential" as used herein may be used with respect to a main longitudinal axis of the device, the cartridge, the housing or the cartridge holder, e.g. the axis which extends through the proximal and distal ends of the cartridge, the cartridge holder or the drug delivery device.

Non-limiting, exemplary embodiments of the invention will now be described with reference to the accompanying drawings, in which:
- Figure 1: shows an embodiment of a drug delivery device;
- Figure 2: schematically illustrates that a user applies torque to a dial grip versus a housing;
- Figures 3a, b: schematically illustrate details of a first embodiment in two different clutch states;
- Figures 4: schematically illustrates a dial encoder;
- Figure 5a-c: schematically illustrate the clutch in different states;
- Figure 6: schematically illustrates details of a second embodiment;
- Figures 7a, b: show half sections of the drug delivery device of Figure 6 in two different clutch states;
- Figure 8a-c: schematically illustrate details of the drug delivery device of Figure 6;
- Figures 9a, b: schematically illustrate details of a third embodiment in two different clutch states;
- Figures 10a, b: schematically illustrate two different clutch states of the device of Figure 9; and
- Figure 11: schematically illustrates a detail of the third embodiment.

In the figures, identical elements, identically acting elements or elements of the same kind may be provided with the same reference numerals.

In the following, some embodiments will be described with reference to an insulin injection device. The present disclosure is however not limited to such application and may equally well be deployed with injection devices that are configured to eject other medicaments or drug delivery devices in general, preferably pen-type devices and/or injection devices.

Embodiments are provided in relation to injection devices, in particular to variable dose injection devices, which record and/or track measurement data on doses delivered thereby. These data may include the size of the selected dose and/or the size of the actually delivered dose, the time and date of administration, the duration of the administration and the like. Features described herein include power management techniques (e.g. to facilitate small batteries and/or to enable efficient power usage).

Certain embodiments in this document are illustrated with respect to the injection device disclosed in EP 2 890 435 B1 where an injection button and grip (dose setting member or dose setter) are combined. The injection button may provide the user interface member for initiating and/or performing a dose delivery operation of the drug delivery device. The grip or knob may provide the user interface member for initiating and/or performing a dose setting operation. These devices are of the dial extension type, i.e. their length increases during dose setting. Other injection devices with the same kinematical behaviour of the dial extension and button during dose setting and dose expelling operational mode are known as, for example, the Kwikpen^{®} device marketed by Eli Lilly and the Novopen^{®} 4 device marketed by Novo Nordisk.

An application of the general principles to these devices therefore appears straightforward and further explanations will be omitted. However, the general principles of the present disclosure are not limited to that kinematical behaviour. Certain other embodiments may be conceived for application to Sanofi's SoloSTAR^{®} injection device where there are separate injection button and grip components / dose setting members. Thus, there may be two separate user interface members, one for the dose setting operation and one for the dose delivery operation. A further example of such a device with a dial grip and a separate button is disclosed in WO 2016/055619 A1.

"Distal" is used herein to specify directions, ends or surfaces which are arranged or are to be arranged to face or point towards a dispensing end of the drug delivery device or components thereof and/or point away from, are to be arranged to face away from or face away from the proximal end. On the other hand, "proximal" is used to specify directions, ends or surfaces which are arranged or are to be arranged to face away from or point away from the dispensing end and/or from the distal end of the drug delivery device or components thereof. The distal end may be the end closest to the dispensing and/or furthest away from the proximal end and the proximal end may be the end furthest away from the dispensing end. A proximal surface may face away from the distal end and/or towards the proximal end. A distal surface may face towards the distal end and/or away from the proximal end. The dispensing end may be the needle end where a needle unit is or is to be mounted to the device, for example.

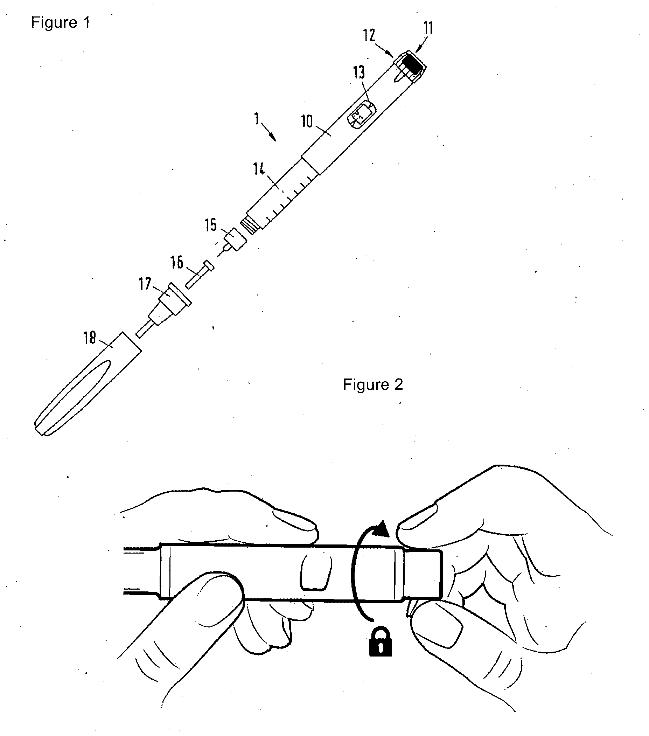

Figure 1 is an exploded view of a medicament delivery device or drug delivery device. In this example, the medicament delivery device is an injection device 1, e.g. a pen-type injector, such an injection pen disclosed in EP 2 890 435 B1 or in EP 2 890 434 B1.

The injection device 1 of Figure 1 is an injection pen that comprises a housing 10 and contains a container 14, e.g. an insulin container, or a receptacle for such a container. The container may contain a drug. A needle 15 can be affixed to the container or the receptacle. The container may be a cartridge and the receptacle may be a cartridge holder. The needle is protected by an inner needle cap 16 and either an outer needle cap 17 or another cap 18. An insulin dose to be ejected from injection device 1 can be set, programmed, or 'dialled in' by turning a dosage knob 12, and a currently programmed or set dose is then displayed via dosage window 13, for instance in multiples of units. The indicia displayed in the window may be provided on a number sleeve or dial sleeve. For example, where the injection device 1 is configured to administer human insulin, the dosage may be displayed in so-called International Units (IU), wherein one IU is the biological equivalent of about 45.5 micrograms of pure crystalline insulin (1/22 mg). Other units may be employed in injection devices for delivering analogue insulin or other medicaments. It should be noted that the selected dose may equally well be displayed differently than as shown in the dosage window 13 in Figure 1.

The dosage window 13 may be in the form of an aperture in the housing 10, which permits a user to view a limited portion of a dial sleeve assembly that is configured to move when the dial grip 12 is turned, to provide a visual indication of a currently set dose. The dial grip 12 is rotated on a helical path with respect to the housing 10 when setting a dose. As shown in Figure 2, dose setting requires that a user applies torque to the dial grip 12 versus the housing 10. A user may be prevented from dialling up after a certain dose value has been selected as will be described in more detail below.

In this example, the dial grip 12 includes one or more formations to facilitate attachment of a data collection device. Especially, the dial grip 12 may be arranged to attach or integrate an electronic (button) module 11 onto the dial grip 12. As an alternative, the dial grip may comprise such a button module of an electronic system.

The injection device 1 may be configured so that turning the dial grip 12 causes a mechanical click sound to provide acoustic feedback to a user. In this embodiment, the dial grip 12 also acts as an injection button. When needle 15 is stuck into a skin portion of a patient, and then dial grip 12 and/or the attached module 11 is pushed in an axial direction, the insulin dose displayed in display window 13 will be ejected from injection device 1. When the needle 15 of injection device 1 remains for a certain time in the skin portion after the dial grip 12 is pushed, the dose is injected into the patient's body. Ejection of the insulin dose may also cause a mechanical click sound, which may be different from the sounds produced when rotating the dial grip 12 during dialing of the dose.

In this embodiment, during delivery of the insulin dose, the dial grip 12 is returned to its initial position in an axial movement, without rotation, while the dial sleeve assembly is rotated to return to its initial position, e.g. to display a dose of zero units. Figure 1 shows the injection device 1 in this OU dialled condition. As noted already, the disclosure is not restricted to insulin but should encompass all drugs in the drug container 14, especially liquid drugs or drug formulations.

Injection device 1 may be used for several injection processes until either the insulin container 14 is empty or the expiration date of the medicament in the injection device 1 (e.g. 28 days after the first use) is reached. In the case of a reusable device, it is possible to replace the insulin container.

Furthermore, before using injection device 1 for the first time, it may be necessary to perform a so-called "prime shot" to remove air from insulin container 14 and needle 15, for instance by selecting two units of insulin and pressing dial grip 12 while holding injection device 1 with the needle 15 upwards. For simplicity of presentation, in the following, it will be assumed that the ejected amounts substantially correspond to the injected doses, so that, for instance the amount of medicament ejected from the injection device 1 is equal to the dose received by the user. Nevertheless, differences (e.g. losses) between the ejected amounts and the injected doses may need to be taken into account.

As explained above, the dial grip 12 also functions as an injection button so that the same component is used for dialling/setting the dose and dispensing/delivering the dose. As an alternative (not shown), a separate injection button may be used which is axially displaceable, at least a limited distance, relative to the dial grip 12 to effect or trigger dose dispensing.

In Figure 1, the electronic module 11 is depicted as being integrated in the proximal end of the injection device 1, specifically integrated into the dial grip/dose button 12. As an alternative, the electronic module 11 may be a separate component part which may be permanently or releasably attached to the injection device 1, e.g. to the grip/dose button 12.

In the following, details of embodiments of a clutch for a drug delivery device as well as a method for operating such a drug delivery device will be described with respect to exemplary embodiments and with reference to Figures 1 to 11. The present disclosure seeks to allow a user to dial to a predetermined value with minimal engagement or thought process. The user can dial a dose as normal, until the limited dose is reached (as determined by the dial encoder), at which point further dialling is prevented (Figure 2). Dialling a dose requires rotation of the internal mechanism relative to the housing, i.e. an outer body. This rotation is generated by the user gripping the housing 10 of the pen and applying torque to the mechanism via the dial grip 12. As depicted in Figure 2, a user may be prevented from dialling up after a certain dose value has been selected. Dialling can be prevented by either: disconnecting the dial grip 12 from the mechanism and/or by locking the dial grip 12 to the housing 10. The user can then proceed to dispense the dose as normal, resetting the mechanism to allow for subsequent doses to be dialled.

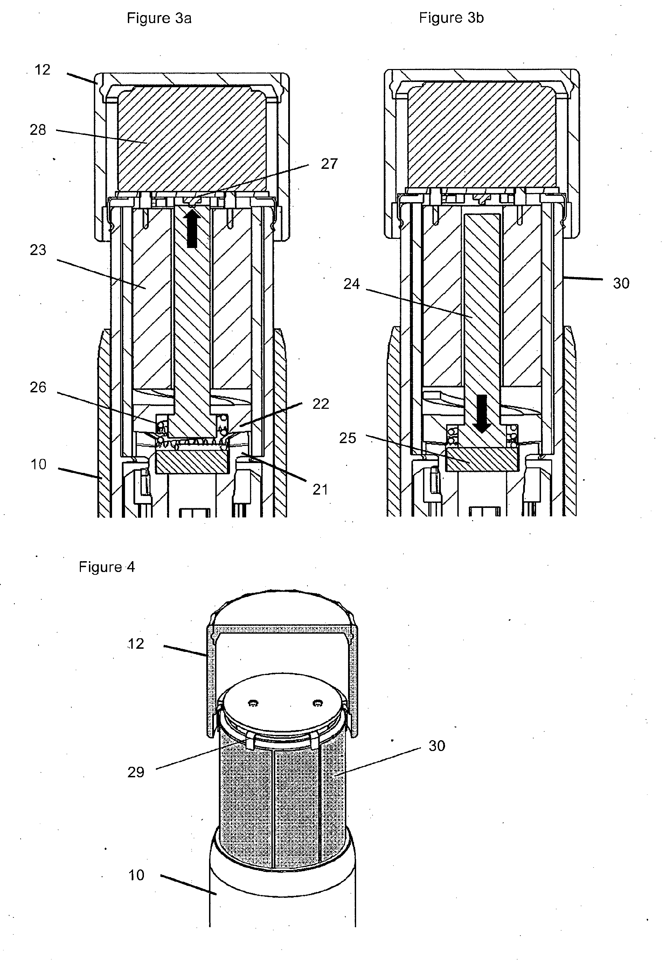

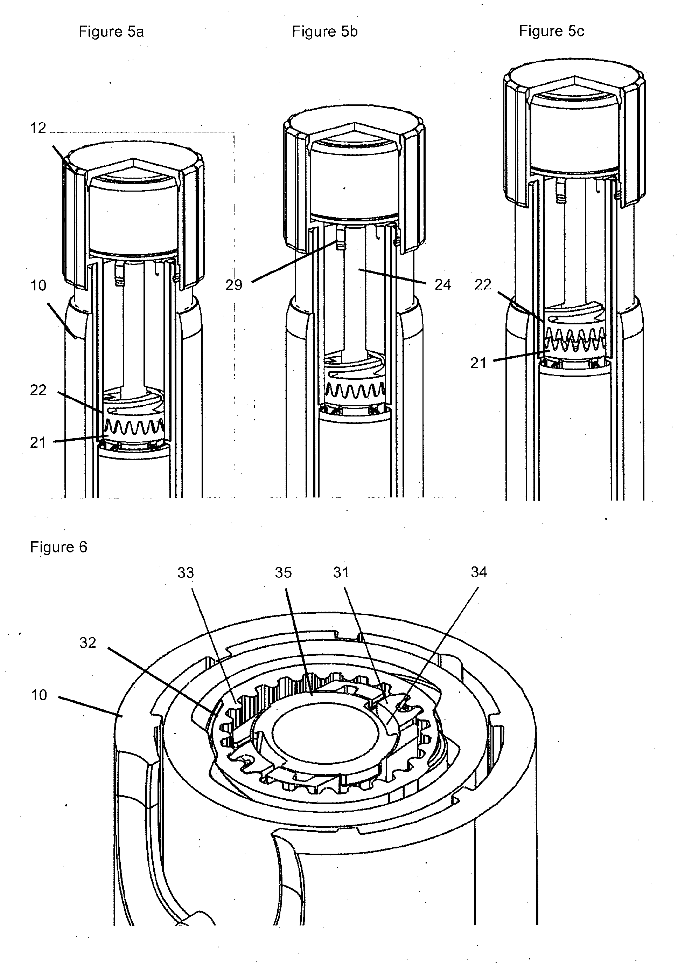

A first exemplary embodiment of a magnetic slip clutch is depicted in Figures 3a to 5c. In this embodiment using the dose setting and drive mechanism as disclosed for an injection pen in EP 2 890 435 B1, the dial grip 12 is connected to the mechanism using a bi-stable clutch 20. This clutch is formed of a first clutch member 21 in the form of a mechanism plate and a second clutch member 22 in the form of a dial plate. As shown in Figures 5a to 5c, the clutch members 21, 22 both comprise a respective ring of clutch teeth, e.g. axially facing clutch teeth facing towards each other as depicted in the Figures. The clutch 20 can be locked into one of two positions or clutch states, namely open or closed. The first clutch member 21 is rotationally constrained to at least one component part of the dose setting and drive mechanism and the second clutch member 22 is rotationally constrained to the dial grip 12.

The first clutch member 21 (mechanism plate) is held in a stable axial position relative to the pen housing 10. The second clutch member 22 (dial plate) is able to move axially between a closed position, where it transfers torque to the mechanism, and an open position, where the two plates are free to slip past each other. Rotating the dial grip 12 can therefore either lead to dialling or slipping depending on the state of the clutch. In other words, if the clutch 20 is closed, torque can be transferred from the dial grip 12 to the dose setting and drive mechanism, whereas if the clutch 20 is open, the dial grip 12 may be rotated relative to the dose setting and drive mechanism without transmitting torque.

The clutch 20 is switched between the two states by powering a latching solenoid actuator (Figure 2). This actuator operates by charging an electromagnetic coil 23 in either polarity for a short time to generate a magnetic field. The field acts to move an actuator pin 24, on which the dial plate (second clutch member 22) is mounted, in either direction.

In the embodiment shown in Figures 3a and 3b, the latching behaviour is generated by a permanent magnet 25 situated within the mechanism plate (first clutch member 21). A spring 26 holds the clutch members 21, 22 apart, while the magnet 25 and magnetizable actuator pin 24 draw them together. Since the spring force is linear, and the magnetic force varies with an inverse square law, a bi-stable system can be created with a switching point. If the clutch members 21, 22 are positioned close together, the magnetic force of magnet 25 will be greater than the spring force, and the clutch members 21, 22 will be drawn closed. If, however, they are in a position further apart than the switching point, they will be held apart by the spring 26. The electromagnet 23 has sufficient strength to move the permanent magnet 25 across the switching point from either direction.

Also shown in Figures 3a and 3b is a detect switch 27, which confirms the clutch location. Also shown is a separate battery 28 for the electromagnetic coil 23. The detect switch 27 is used to monitor the position of the second clutch member 22 (dial plate). This switch 27 is positioned such that the clutch teeth cannot pass one another without the switch 27 closing. If the dial grip 12 is rotating and the detect switch 27 is open, this will be counted as dialled units. If the dial grip 12 is rotating, but the detect switch 27 is closed, the processor will deduce that the clutch 20 is slipping.

In an alternative embodiment (not depicted) the permanent magnet 25 could be replaced by a detent or snap feature which is overcome in both directions by the solenoid 23 and provides two stable positions.

The clutch 20 may be switched from its closed state (Figure 3b) into its open state (Figure 3a) by conducting the following steps: First, the coil 25 is powered with energy from the battery 28 by means of the processor, thus generating a magnetic field which imparts a force on the actuator pin 24, pulling it away from the permanent magnet 25. If sufficient separation is achieved so that spring 26 force exceeds the magnetic force of magnet 25, the coil 23 can be depowered. Then, the spring 26 pushes clutch plate 22 into the open position such that the clutch teeth are fully disengaged (or only partially engaged), allowing for slipping of the clutch 20. The clutch 20 may be switched from its open state (Figure 3a) into its closed state (Figure 3b) by conducting the following steps: First, the coil 23 is powered with opposite polarity generating a magnetic field which imparts a force on the actuator pin 24, pushing it towards the permanent magnet 25. If sufficient separation is achieved the spring force becomes smaller than the magnetic force and the coil 23 can be depowered. Then the magnet 25 pulls the actuator pin 24 into contact such that the clutch teeth are fully engaged, transferring all torque from the dial grip 12 to the mechanism.

In this embodiment, an encoder is used as a sensor to detect relative rotation between the dial grip 12 and a portion of the button assembly which is rotationally splined to the housing 10. The dial encoder is formed of sprung PCB contacts 29 and conductive strips 30 on said portion of the button assembly. Relative motion causes the strips 30 to connect different combinations of PCB contacts 29. With this encoder information, the processor can trigger a switch of the state of clutch 20 when needed, i.e. if a preselected or predefined maximum dose value is dialled.

If slipping is detected by switch 27 below the maximum dose limit, the electromagnet 23 may be charged to engage the latching clutch 20. When the maximum dose limit is reached, the electromagnet 23 will be charged to release the latch, allowing the clutch 20 to slip.

In Figures 5a to 5c the cutch is shown engaged at the start of dialling (Figure 5a) and remains engaged (Figure 5b) until the limit dose is encoded, at which point the clutch 20 will allow slipping (Figure 5c). Figure 5 further shows the dial extension, i.e. the dial grip 12 moving away from the housing 10 as the dose is increased.

A OU switch may also be required to reset the dialled dose count and commit the dose record to memory. This can be achieved by shorting all the conductive strips 30 in the OU (button pressed) position.

A second exemplary embodiment of a magnetic clutch locking a dial clicker 31 is depicted in Figures 6 to 8c. In this embodiment, again using the dose setting and drive mechanism as disclosed for an injection pen in EP 2 890 435 B1 or in EP 2 890 434 B1, the dial grip 12 is mechanically splined to the mechanism. The mode of operation is to lock the mechanism via the dial clicker 31 to an inner housing 32 which is rotationally constrained to the stationary housing 10. The dial clicker 31 is formed by one or more arms (Figure 6 shows two arms) which can deflect radially. The inner housing 32 is provided with a series of axially extending splines 33 on an inner surface of the inner housing 32.

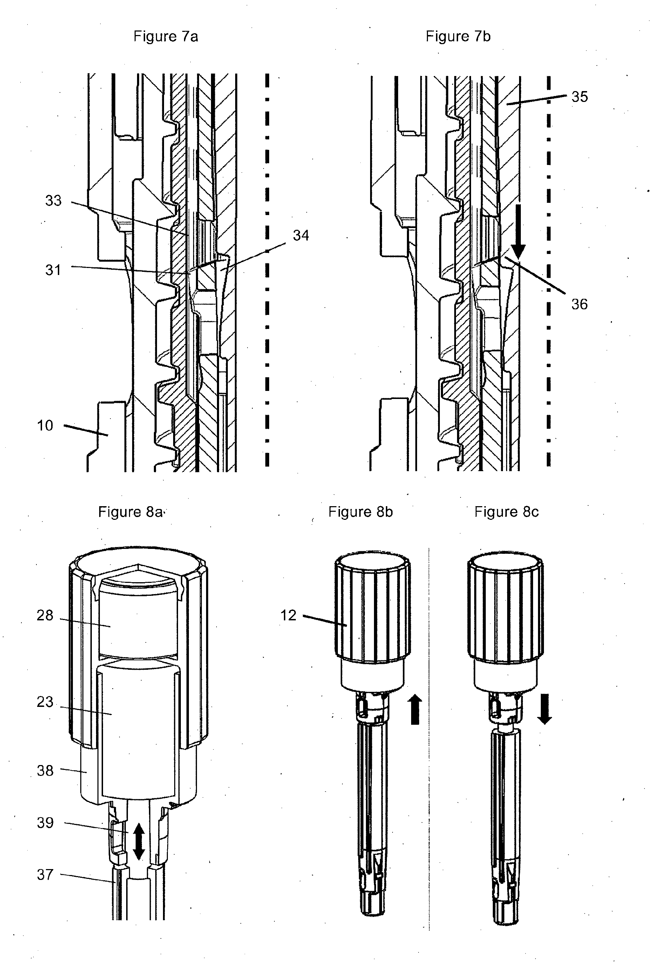

The arms of the dial clicker 31 engage the splines 33 of the inner housing 32 but may jump from one spline 33 to the next spline 33 by deflecting inwardly as relative rotation occurs between the dial clicker 31 and the inner housing 32. This deflection is permitted due to recesses 34 in button component 35 (Figures 6 and 7a). In other words, in normal use, the dial clicker 31 arms flex inward over splines 33 on the inner housing 32 on each unit of drug dialled, allowing for relative rotation. The button component 35 and the clicker 31 arms are designed so that when the button component 35 is pressed down, e.g. during dose dispensing, the arms are blocked from flexing as a blocking portion 36 of the button component 35 is located radially inwards of the arms (Figure 7b). In other words, depending on the relative axial position of the recess 34 or the blocking portion 36 of the button component 35 with respect to the arms of clicker 31, relative rotation of the clicker 31 with respect to the inner housing 32 is either permitted or prevented.

In this embodiment the button component 35 is split into a lower portion which is an engagement portion 37 and an upper portion which is a trigger portion 38 which are axially movable for a limited distance relative to each other by means of the clutch actuator. In more detail, the lower portion 37 can be moved down into a blocking position in advance of the upper portion 38. That is to say, dialling can be blocked prior to the button being pressed.

A bi-stable solenoid actuator (electromagnetic coil 23 and actuator pin 39), similar to that described in the first embodiment, is included in the button subassembly (Figures 8a to 8c). For example, the section of button subassembly (Figure 8a) shows the rechargeable battery 28, the solenoid coil 23, the upper portion 38 of button component 35, the actuator pin 39, and the lower portion 37 of button component 35. In this way, the button effectively has two lengths. In the shorter configuration, the button acts in the same manner as the unmodified device, and the user can dial and dispense as normal. In the longer configuration, the user is unable to dial, and must proceed to dispense the dose.

In use, the solenoid clutch actuator 23, 39 will begin in the short configuration (Figure 8b) and remain there until the encoder observes that the limit dose has been reached. At this point, the solenoid clutch actuator 23, 39 will be switched, driving the button 35 into the longer state (Figure 8c). The upper portion 38 of button component 35 will be prevented from moving upward, meaning the lower portion 37 of button component 35 must move down and block the dial clicker 31 arms, as shown in Figure 7b. The dial grip 12 will then feel locked to the user. Pushing down on the button will dispense the dose and collapse the solenoid back into the shorter state. Therefore, once the dose is complete and the button is released, the dial clicker 31 arms will be unblocked once more and further doses can be dialled.

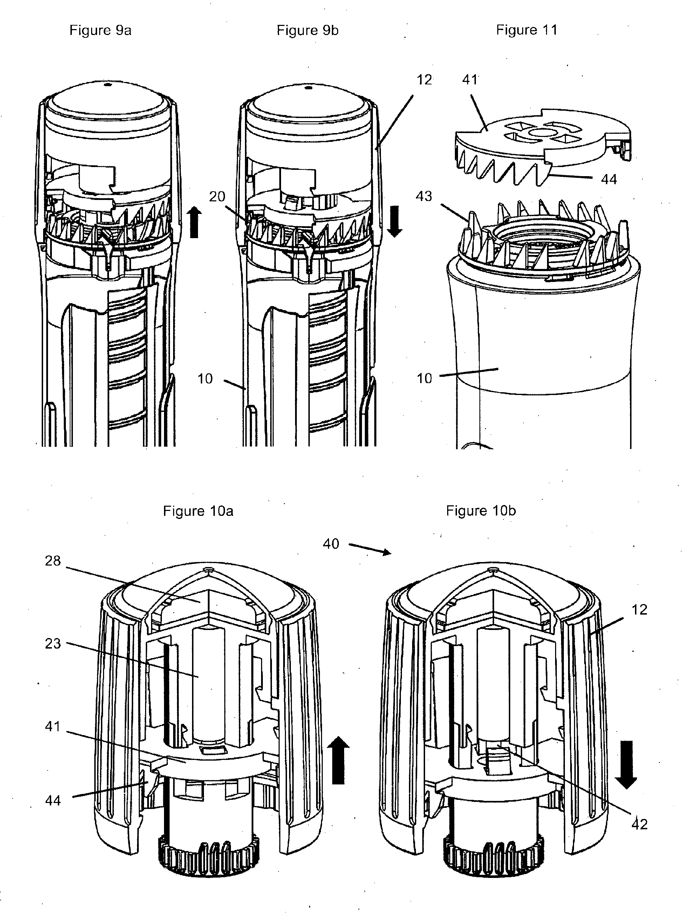

A third exemplary embodiment of a magnetic clutch locking a dose button 40 is depicted in Figures 9a to 11. In this embodiment, using the dose setting and drive mechanism as disclosed for an injection pen in WO 2016/055619 A1, the dial grip 12 is mechanically splined to the mechanism. A sliding collar 41 is provided axially constrained to an actuator pin 42 and rotationally constrained to the button 40. The mode of operation is to use a sliding collar 41 to engage or disengage a locking clutch 20 between the mechanism and the housing 10. For this purpose, first and second clutch members 43, 44 are provided on the sliding collar 41 and on the housing 10 in the form of respective rings of teeth.

This embodiment is best applied to devices without dial extension (as in WO 2016/055619 A1) so that the button 40 and the housing 10 are at a fixed separation throughout dialling. Again, a bi-stable solenoid clutch actuator (electromagnetic coil 23, actuator pin 42) is employed to provide the motion to switch the configuration of the button subassembly once the maximum encoder value is read. Figures 9a and 10a show the clutch in an open state permitting rotation between button 40 and housing 10, whereas Figures 9b and 10b show the clutch in a closed state preventing rotation between button 40 and housing 10.

In use, the solenoid clutch actuator 23, 42 will begin in the short configuration and hold the collar 41 in clearance with the housing 10. In this configuration dialling proceeds as with an unmodified device. Once the encoder reads that the limit dose has been reached, the solenoid clutch actuator 23, 42 will be switched, driving the collar 41 into contact with the housing 10.

The clutch teeth will prevent further dialling but may be asymmetrically ramped to allow for dialling back down (Figure 11). This ramp would serve to overcome the bi-stable point of the solenoid clutch actuator 23, 42, switching it back to the shorter configuration on a dialling down. In other words, the ramped clutch teeth enable downward dialling and to reset the clutch/solenoid position.

As with the second embodiment, pushing down on the button 40 will dispense the dose and collapse the solenoid clutch actuator 23, 42 back into the shorter state. Therefore, once the dose is complete and the button 40 is released, the clutch will be open allowing further doses to be dialled.

Although described mainly with respect to a drug delivery device having a similar working principle as the device disclosed in EP 2 890 435 B1, EP 2 890 434 B1 or WO 2016/055619 A1, the electronic clutch is applicable to any other type of drug delivery device having component parts performing a relative axial and/or rotational movement in defined conditions or states.

### Reference Numerals

- 1: drug delivery device
- 10: housing
- 11: button module
- 12: dial grip
- 13: dosage window
- 14: container/container receptacle
- 15: needle
- 16: inner needle cap
- 17: outer needle cap
- 18: cap
- 20: clutch
- 21: first clutch member (mechanism plate)
- 22: second clutch member (dial plate)
- 23: electromagnetic coil
- 24: actuator pin
- 25: permanent magnet
- 26: spring
- 27: switch
- 28: power source (battery)
- 29: contact
- 30: conductive strip
- 31: dial clicker with arm(s)
- 32: inner housing
- 33: spline
- 34: recess
- 35: button
- 36: blocking feature
- 37: lower or engagement portion
- 38: upper or trigger portion
- 39: actuator pin
- 40: button
- 41: clutch collar
- 42: actuator pin
- 43: first clutch member
- 44: second clutch member

## Claims

1. A drug delivery device (1) comprising
- a housing (10; 32),
- a dial grip (12) manually operable by a user for setting an individual dose to be delivered by the drug delivery device (1),
- a dose setting and drive mechanism, which is configured to perform a dose setting operation in response to the operation of the dial grip (12) and which is configured to perform a dose delivery operation for delivering the set dose,
- a clutch (20) comprising two clutch members (21, 22; 31, 33; 43, 44) for rotationally coupling and de-coupling the dial grip (12) to the housing (10; 32) and/or to the dose setting and drive mechanism,
- at least one processor,
- a sensor arrangement (29, 30) connected to the at least one processor and operable to generate measurement data indicative of the dose setting operation, and
- a power source (28) connected to the at least one processor,
**characterized in that** the drug delivery device (1) further comprises a clutch actuator (23, 24; 23; 39, 23, 42) operable by the at least one processor to trigger a switch of the state of the clutch (20),
wherein the at least one processor is configured to operate the clutch actuator (23, 24; 23; 39, 23, 42) if the measurement data generated by the sensor arrangement (29, 30) reaches a threshold.

2. The drug delivery device (1) according to claim 1, wherein the clutch actuator (23, 24; 23; 39, 23, 42) comprises an electrical drive for translating or locking at least one of the clutch members (21, 22; 31, 33; 43, 44).

3. The drug delivery device (1) according to claim 2, wherein the clutch actuator (23, 24; 23; 39, 23, 42) is a solenoid actuator comprising at least one electromagnet coil (23) and an actuator pin (24; 39; 42).

4. The drug delivery device (1) according to claim 3, wherein the actuator pin (24; 39; 42) is secured to one of the clutch members (22; 43).

5. The drug delivery device (1) according to any one of claims 3 or 4, wherein the at least one electromagnet coil (23) at least partially surrounds the actuator pin (24; 39; 42) such that charging the at least one electromagnet coil (23) with a first polarity generates a force biasing the actuator pin (24; 39; 42) in a first direction, whereas charging the at least one electromagnet coil (23) with a second polarity generates a force biasing the actuator pin (24; 39; 42) in a second direction opposite to the first direction.

6. The drug delivery device (1) according to any one of claims 1 to 5, wherein the clutch actuator (23, 24; 23; 39, 23, 42) further comprises a permanent magnet (25) secured on at least one of the clutch members (21).

7. The drug delivery device (1) according to claims 3 to 5 and 6, wherein the permanent magnet (25) is arranged such that it exerts a magnetic force on the actuator pin (24) biasing the clutch members (21, 22) into contact.

8. The drug delivery device (1) according to any one of the preceding claims, wherein the clutch actuator (23, 24; 23; 39, 23, 42) further comprises a spring (26) biasing the clutch members (21, 22) in opposite directions.

9. The drug delivery device (1) according to any one of the preceding claims, wherein
- the dose setting and drive mechanism is rotationally fixed to a first ring of teeth forming a first clutch member (21),
- the dial grip (12) is rotationally fixed to a second ring of teeth forming a second clutch member (22),
- the actuator pin (24) is axially fixed to the second ring of teeth (22),
such that transmission of torque from the dial grip (12) to the dose setting and drive mechanism occurs with the clutch (20) coupled by engagement of the first ring of teeth (21) with the second ring of teeth (22) whereas transmission of torque from the dial grip (12) to the dose setting and drive mechanism is prevented with the clutch (20) de-coupled by disengagement of the first ring of teeth (21) with the second ring of teeth (22).

10. The drug delivery device (1) according to any one of claims 1 to 8, further comprising
- a stationary inner housing (32) with splines (33) forming a first clutch member,
- a dial clicker (31) rotationally fixed to the dial grip (12), the dial clicker (31) comprising at least one elastically deflectable arm for engaging the splines (33) of the inner housing (32), the clicker (31) arm forming a second clutch member, and
- a button (35) axially displaceable relative to the dial clicker (31) to perform the dose delivery operation for delivering the set dose, the button (35) comprising a trigger portion (38) and an engagement portion (37), the engagement portion (37) comprising a recess (34) for receiving the elastically deflectable arm and axially spaced from the recess (34) a blocking feature (36) preventing deflection of the elastically deflectable arm, wherein the actuator pin (39) is axially fixed to the engagement portion (37)
such that dose setting operation of the dial grip (12) is permitted with the clutch de-coupled by positioning the recess (34) such that deflection of the elastically deflectable arm is permitted, whereas dose setting operation of the dial grip (12) is blocked with the clutch coupled by preventing deflection of the elastically deflectable arm.

11. The drug delivery device (1) according to claim 10, wherein the button (35) is a multi-component part with the trigger portion (38) axially displaceable relative to the engagement portion (37) by means of the actuator pin (39).

12. The drug delivery device (1) according to any one of claims 1 to 8, wherein
- the housing (10) is rotationally fixed to a first ring of teeth forming a second clutch member (43),
- the dial grip (12) is rotationally fixed to a second ring of teeth forming a first Clutch member (44),
- the actuator pin (42) is axially fixed to the second ring of teeth (44),
such that dose setting operation of the dial grip (12) is permitted with the clutch de-coupled by disengaging the first ring of teeth (43) from the second ring of teeth (44), whereas dose setting operation of the dial grip (12) is blocked with the clutch coupled by engaging the first ring of teeth (43) with the second ring of teeth (44).

13. The drug delivery device (1) according to any one of the preceding claims, wherein the clutch actuator (23, 24; 23; 39, 23, 42) is connected to an additional power source.

14. The drug delivery device (1) according to any one of the preceding claims, further comprising a container receptacle (14) which is permanently or releasably connected to the dose setting and drive mechanism and which is adapted to receive a container containing a medicament.

15. A method for operating a drug delivery device (1), the drug delivery device (1) comprising
- a housing (10),
- a dial grip (12) manually operable by a user for setting an individual dose to be delivered by the drug delivery device (1),
- a dose setting and drive mechanism, which is configured to perform a dose setting operation in response to the operation of the dial grip (12) and which is configured to perform a dose delivery operation for delivering the set dose,
- a clutch (20) comprising two clutch members (21, 22; 31, 33; 43, 44) for rotationally coupling and de-coupling the dial grip (12) to the housing (10) and/or to the dose setting and drive mechanism,
- at least one processor,
- a sensor arrangement (29, 30) connected to the at least one processor and operable to generate measurement data indicative of the dose setting operation, and
- a power source (28) connected to the at least one processor, and
- a clutch actuator (23, 24; 23; 39, 23, 42) operable by the at least one processor to trigger a switch of the state of the clutch (20),
wherein the method comprises the following steps:
a) setting an individual dose by manual operation of the dial grip (12),
b) generating measurement data indicative of the dose setting operation by the sensor arrangement (29, 30),
c) operating the clutch actuator (23, 24; 23; 39, 23, 42) if the measurement data generated by the sensor arrangement (29, 30) reaches a threshold, thereby locking the dial grip (12) or disengaging the dial grip (12) from the dose setting and drive mechanism,
d) performing a dose delivery operation for delivering the set dose.
